# EUROPEAN PATENT SPECIFICATION

(11) **EP 1 987 153 B1**
(45) Date of publication and mention of the grant of the patent: **19.01.2011**
(21) Application number: 07702492.5
(22) Date of filing: 15.02.2007
(51) Int. Cl.: C12P 19/26

(54) **PRODUCTION OF LOW MOLECULAR WEIGHT HYALURONIC ACID**
PRODUKTION VON HYALURONSÄURE MIT GERINGEM MOLEKULARGEWICHT
PRODUCTION D'ACIDE HYALURONIQUE DE BAS POIDS MOLÉCULAIRE

(30) Priority: 15.02.2006 DK 200600218
(43) Date of publication of application: 05.11.2008
(73) Proprietor: Novozymes Biopharma DK A/S, 2880 Bagsvaerd (DK)
(72) Inventor: STOCKS, Stuart, M., DK-1307 Copenhagen K (DK); BROWN, Stephen, Davis, CA 95616 (US)
(74) Representative: Lindum, Peter Würtz
(86) International application number: PCT/DK2007/000074
(87) International publication number: WO 2007/093179

(56) References cited:
- ARMSTRONG ET AL: "Culture conditions affect the molecular weight properties of hyaluronic acid produced by Streptococcus zooepidemicus" APPLIED AND ENVIRONMENTAL MICROBIOLOGY, vol. 63, 1997, pages 2759-2764, XP002436439 cited in the application
- WIDNER ET AL: "Hyaluronic acid production in Bacillus subtilis" APPLIED AND ENVIRONMENTAL MICROBIOLOGY, vol. 71, 2005, pages 3747-3752, XP002424013
- KIM ET AL: "A novel approach to the production of hyaluronic acid by Streptococcus zooepidemicus" JOURNAL OF MICROBIOLOGY AND BIOTECHNOLOGY, vol. 16, December 2006 (2006-12), pages 1849-1855, XP008079697

## Description

### FIELD OF THE INVENTION

The present invention relates to methods for the recombinant production in a Bacillus host cell of hyaluronic acid (HA or hyaluronan) with a low average molecular weight (MW) by temperature-controlled fermentation. The HA-producing host cell is first fermented at a temperature conducive for its growth, followed by a shift to a higher temperature favourable for production of HA of the desired low MW. The temperature and pH favourable for low-MW HA-production may in some instances even lie outside the ranges of pH and temperature usually considered favourable for growth of the microorganism being fermented.

### BACKGROUND OF THE INVENTION

The most abundant heteropolysaccharides of the body are the glycosaminoglycans. Glycosaminoglycans are unbranched carbohydrate polymers, consisting of repeating disaccharide units (only keratan sulphate is branched in the core region of the carbohydrate). The disaccharide units generally comprise, as a first saccharide unit, one of two modified sugars - N-acetylgalactosamine (GaINAc) or N-acetylglucosamine (GIcNAc). The second unit is usually an uronic acid, such as glucuronic acid (GlcUA) or iduronate.

Glycosaminoglycans are negatively charged molecules, and have an extended conformation that imparts high viscosity when in solution. Glycosaminoglycans are located primarily on the surface of cells or in the extracellular matrix. Glycosaminoglycans also have low compressibility in solution and, as a result, are ideal as a physiological lubricating fluid, e.g., joints. The rigidity of glycosaminoglycans provides structural integrity to cells and provides passageways between cells, allowing for cell migration. The glycosaminoglycans of highest physiological importance are hyaluronan, chondroitin sulfate, heparin, heparan sulfate, dermatan sulfate, and keratan sulfate. Most glycosaminoglycans bind covalently to a proteoglycan core protein through specific oligosaccharide structures. Hyaluronan forms large aggregates with certain proteoglycans, but is an exception as free carbohydrate chains form non-covalent complexes with proteoglycans.

Hyaluronic acid is defined herein as an unsulphated glycosaminoglycan composed of repeating disaccharide units of N-acetylglucosamine (GlcNAc) and glucuronic acid (GlcUA) linked together by alternating beta-1,4 and beta-1,3 glycosidic bonds. Hyaluronic acid is also known as hyaluronan, hyaluronate, or HA. The terms hyaluronan and hyaluronic acid are used interchangeably herein.

Numerous roles of hyaluronan in the body have been identified (see, Laurent T. C. and Fraser J. R. E., 1992, FASEB J. 6: 2397-2404; and Toole B.P., 1991, "Proteoglycans and hyaluronan in morphogenesis and differentiation." In: Cell Biology of the Extracellular Matrix, pp. 305-341, Hay E. D., ed., Plenum, New York). Hyaluronan is present in hyaline cartilage, synovial joint fluid, and skin tissue, both dermis and epidermis. Hyaluronan is also suspected of having a role in numerous physiological functions, such as adhesion, development, cell motility, cancer, angiogenesis, and wound healing. Due to the unique physical and biological properties of hyaluronan, it is employed in eye and joint surgery and is being evaluated in other medical procedures. Products of hyaluronan have also been developed for use in orthopaedics, rheumatology, and dermatology.

Rooster combs are a significant commercial source for hyaluronan. Microorganisms are an alternative source. U.S. Patent No. 4,801,539 discloses a fermentation method for preparing hyaluronic acid involving a strain of Streptococcus zooepidemicus with reported yields of about 3.6 g of hyaluronic acid per liter. European Patent No. EP0694616 discloses fermentation processes using an improved strain of Streptococcus zooepidemicus with reported yields of about 3.5 g of hyaluronic acid per liter.

The microorganisms used for production of hyaluronic acid by fermentation are strains of pathogenic bacteria, foremost among them being several Streptococcus spp. The group A and group C streptococci surround themselves with a nonantigenic capsule composed of hyaluronan, which is identical in composition to that found in connective tissue and joints. Pasteurella multocida, another pathogenic encapsulating bacteria, also surrounds its cells with hyaluronan.

Hyaluronan synthases have been described from vertebrates, bacterial pathogens, and algal viruses (DeAngelis, P. L., 1999, Cell. Mol. Life Sci. 56: 670-682). WO 99/23227 discloses a Group I hyaluronate synthase from Streptococcus equisimilis. WO 99/51265 and WO 00/27437 describe a Group II hyaluronate synthase from Pasturella multocida. Ferretti et al. disclose the hyaluronan synthase operon of Streptococcus pyogenes, which is composed of three genes, hasA, hasB, and hasC, that encode hyaluronate synthase, UDP glucose dehydrogenase, and UDP-glucose pyrophosphorylase, respectively (Proc. Natl. Acad. Sci. USA. 98, 4658-4663, 2001). WO 99/51265 describes a nucleic acid segment having a coding region for a Streptococcus equisimilis hyaluronan synthase.

The production of hyaluronic acid, particularly of low average molecular weight, such as, below 1 MDa, is most commonly achieved by initially isolating higher molecular weight material (>1MDa) from fermentation broth or from animal sources. The desired reduction in molecular weight is then achieved, typically, through fractionation, by mechanical/physical means, or by chemical means.

Fractionation has been done over a size-selective membrane with the resulting fractions having an average molecular weight in the range of 30,000 - 730,000 Dalton, larger molecules being retained by the membrane. Solvent precipitation is also well-established, the larger molecules are precipitated first, but this method lacks the resolution of membrane fractionation. In general, fractionation methods tend to be favourable for the isolation of larger molecules, and not really suitable for the production of small molecules.

Using mechanical means, the molecules are subjected to a shear stress sufficient to cause breakage. For example, HA material of 1,700,000 Da can be reduced to below 500,000 Da in a high pressure homogeniser (W09104279-A). The method can be scaled up with, for example, machines of the Manton-Gaulin type, these being available at various scales and capable of processing material at rates of 10 1/h up to the order of several m³/h.

Physical means, such as exposure to ultrasound, have also been reported to work, but it is difficult to implement such methods at anything much larger than the laboratory scale (Orvisky et al. 1993. Size exclusion chromatographic characterization of sodium hyaluronate fractions prepared by high energetic sonication. Chromatographia vol. 37 (1-2): 20-22).

Chemical means, such as hydrolysis at the extremes values of pH, have also been described, or in the presence of other chemicals. Such methods might be unsuitable if the pH mediator or chemical must not be present in the final product, or if fine control is needed to start and stop the process; mixing rates might be limiting in large scale.

When isolating HA from animal sources, there is no control over the starting MW, it is almost always of high order (>5Da). Fermented HA from wildtype microorganisms most commonly has a lower MW than from animal sources, but still higher than 1 Mda.

HA from wildtype *Streptococcus* fermentations has often been quoted as having an average molecular weight of in the range of 1.5 MDa to 3.2 MDa. A *Streptococcus zooepidemicus* which was grown in a setup where it had a maximum specific growth rate at 40°C, was reported to produce hyaluronic acid of increasingly higher molecular weight when the fermentation temperature was reduced from 40°C to 32°C. This was suggested to be the result of a decreasing specific growth rate (Armstrong & Johns. 1997. Appl. Envir. Microbiol. vol. 63: 2759-2764). Other authors have confirmed a correlation between the specific growth rate of S. *zooepidemicus* and its HA productivity as well as the molecular weight of the HA it produces (Chong B.F., et al. 2005. Microbial hyaluronic acid production. Appl. Microbiol. and Biotech. vol. 66(4): 341-351). However, the literature on the subject of microbial HA production is altogether focused on maximising the molecular weight of the HA, not reducing it.

HA production in Bacillus subtilis has also been disclosed (Widner et al. 2005. Appl. Envir. Microbiol. vol 71 : 3747-3752).

Bacilli are well established as host cell systems for the production of native and recombinant proteins, including recombinant expression of exogenous hyaluronan synthase enzymes which enable the host cell to produce hyaluronic acid (WO 2003054163). It is an object of the present invention to provide methods for producing a hyaluronic acid with a desired low average molecular weight in the range of **300,000** - 800,000 Dalton in a recombinant *Bacillus* host cell.

### SUMMARY OF THE INVENTION

As mentioned above, the production of hyaluronic acid with a low average molecular weight, such as, below 800,000 Da, is most commonly achieved by first isolating higher MW material (>1MDa) and then reducing the molecular weight, typically, through fractionation, by mechanical/physical means, or by chemical means.

The present inventions provides a fermentation method with a recombinant host cell that directly produces HA having the desired low average MW of less than 800,000 Da which in turn provides numerous down stream processing benefits.

When a HA material is produced directly having a close-to-desired low MW, then each step of the production process, including fermentation and the unit operations of recovery, benefits from a lower viscosity. In addition, it becomes possible to operate at a higher overall HA concentration than with molecules of a higher MW. This releases production capacity, allows a faster throughput, and results in a more efficient process that is more readily controlled. There are also benefits in quality control.

Accordingly, in a first aspect the invention relates to a method for producing a hyaluronic acid with a desired average molecular weight in the range of **300,000** - 800,000 Dalton, the method comprising the steps of:
(a) cultivating a recombinant *Bacillus* host cell at a first temperature conducive to its growth, wherein the *Bacillus* host cell comprises a nucleic acid construct comprising a hyaluronan synthase encoding sequence operably linked to a promoter sequence foreign to the hyaluronan synthase encoding sequence;
(b) then cultivating the recombinant *Bacillus* host cell of step (a) at a second temperature higher than the first temperature of step (a) under conditions suitable for production of the hyaluronic acid, where the second temperature is in the range 40°C - 52°C and at least 5°C higher than the first temperature, whereby the *Bacillus* host cell produces hyaluronic acid with a desired average molecular weight in the range selected from the group of molecular weight ranges consisting of 300 - 400 kDa, 400 - 500 kDa, 500 - 600 kDa, 600 - 700 kDa, 700 - 800 kDa; and (c)recovering the hyaluronic acid.

### BRIEF DESCRIPTION OF DRAWINGS

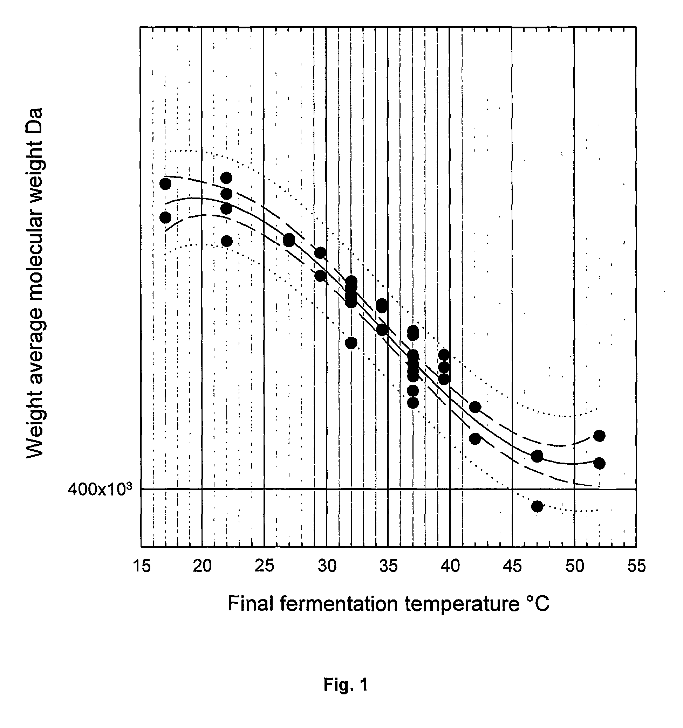

Figure 1 shows the trend for the average molecular weight at the end of fermentations as a function of the final fermentation temperature, as determined by GPC-MALLS. The figure shows that a desired MW can be selected through manipulation of the fermentation temperature. There is a maximum at low final temperatures of 17°C, and a minimum at high fermentation temperatures of 52°C. The identity of the true maximum has been protected by selecting a non-zero origin for molecular weight.

### DETAILED DESCRIPTION OF THE INVENTION

The present invention relates to methods for producing a hyaluronic acid with a desired average molecular weight in the range of 300,000 - 800,000 Dalton, the methods comprising the steps of:
(a) cultivating a recombinant *Bacillus* host cell at a first temperature conducive to its growth, wherein the *Bacillus* host cell comprises a nucleic acid construct comprising a hyaluronan synthase encoding sequence operably linked to a promoter sequence foreign to the hyaluronan synthase encoding sequence;
(b) then cultivating the recombinant *Bacillus* host cell of step (a) at a second temperature higher than the first temperature of step (a) under conditions suitable for production of the hyaluronic acid, where the second temperature is in the range 40°C - 52°C and at least 5°C higher than the first temperature, whereby the *Bacillus* host cell produces hyaluronic acid with a desired average molecular weight in the range selected from the group of molecular weight ranges consisting of 300 - 400 kDa, 400 - 500 kDa, 500 - 600 kDa, 600 - 700 kDa, 700 - 800 kDa; and (c)recovering the hyaluronic acid.

The methods of the present invention represent an improvement over the production of hyaluronan from pathogenic, encapsulating bacteria with subsequent process steps to reduce the molecular weight. In encapsulating bacteria, a large quantity of the hyaluronan is produced in the capsule. In processing and purifying hyaluronan from such sources, it is first necessary to remove the hyaluronan from the capsule, such as by the use of a surfactant, or detergent, such as SDS. This creates a complicating step in commercial production of hyaluronan, as the surfactant must be added in order to liberate a large portion of the hyaluronan, and subsequently the surfactant must be removed prior to final purification.

The present invention allows the production of a large quantity of a low-MW hyaluronan, which is produced in a safe non-encapsulating host cell, as free hyaluronan.

Since the hyaluronan of the recombinant *Bacillus* cell is expressed directly to the culture medium, a simple process may be used to isolate the hyaluronan from the culture medium. First, the *Bacillus* cells and cellular debris are physically removed from the culture medium. The culture medium may be diluted first, if desired, to reduce the viscosity of the medium. Many methods are known to those skilled in the art for removing cells from culture medium, such as centrifugation or microfiltration. If desired, the remaining supernatant may then be filtered, such as by ultrafiltration, to concentrate and remove small molecule contaminants from the hyaluronan. Following removal of the cells and cellular debris, a simple precipitation of the hyaluronan from the medium is performed by known mechanisms. Salt, alcohol, or combinations of salt and alcohol may be used to precipitate the hyaluronan from the filtrate. Once reduced to a precipitate, the hyaluronan can be easily isolated from the solution by physical means. Alternatively, the hyaluronan may be dried or concentrated from the filtrate solution by using evaporative techniques known to the art, such as spray drying.

The methods of the present invention thus represent an improvement over existing techniques for commercially producing hyaluronan by fermentation, in not requiring the use of a surfactant in the purification of hyaluronan from cells in culture.

In the methods of the invention, the *Bacillus* host is culitvated at a first temperature conducive to its growth in order to build up a large amount of active biomass for the subsequent HA synthesis. Bacilli are capable of growth in a wide range of temperatures, proviced there are no other limiting factors. For instance, in rich culture media, it must be ensured that there is sufficient aeration at higher temperatures to achieve a high specific growth rate.

Accordingly, a preferred embodiment relates to the method of the first aspect, wherein the first temperature is in the range of 10°C - 60°C, preferably 20°C - 50°C, and more preferably in the range of 30°C - 45°C, most preferably in the range of 34°C - 40°C.

Once the desired amount of biomass has been established, the *Bacillus* host is cultivated at a second temperature which is set at least 5°C higher than the first temperature during biomass build-up, and under conditions suitable for production of the hyaluronic acid. Precisely how much higher the second temperature is set, depends on the desired MW of the HA to be produced. The lower the desired MW is, the higher the temperature must be set.

So, the second temperature is in the range of 40°C - 52°C. Naturally, the preferred ranges of the first cultivating temperature are to be combined with the suitable preferred ranges of the second cultivating temperature in the method of the invention.

The second temperature is at least 5°C higher than the first temperature, preferably the second temperature is at least 6°C, 7°C, 8°C, 9°C, 10°C, 11°C, 12°C, 13°C, 14°C, 15°C, 16°C, 17°C, 18°C, 19°C, 20°C, 21 °C, 22°C, 23°C, 24°C, 25°C, 26°C, 27°C, 28°C, 29°C, and most preferably at least 30°C higher than the first temperature.

The duration of the cultivating step at the first temperature in the methods of the invention, where biomass is built up, depends on a number of factors, including the culturing conditions, the fermentation volume, the particular *Bacillus* strain chosen etc., and of course also the first temperature. The duration of the cultivating step at the second temperature, which is when the low-MW HA is produced, also depends on different factors. Consequently, the total cultivating time which is defined as the duration of both cultivating steps, is not easily determined. However, in a preferred embodiment, the cultivating step at the second temperature takes up at least 20% of the total cultivating time, preferably the cultivating step at the second temperature takes up at least 30%, 40%, 50%, 60%, 70%, 80%, or most preferably at least 90% of the total cultivating time.

A preferred embodiment relates to the method of the first aspect, wherein the the second temperature is sufficiently higher than the first temperature to allow the *Bacillus* host cell to produce hyaluronic acid with a desired average molecular weight in a range selected from the group of molecular weight ranges consisting of **300 - 350 kDa, 350 - 400 kDa, 400** - **450 kDa, 450 - 500 kDa, 500 - 550 kDa, 550 - 600 kDa, 600 - 650 kDa, 650 - 700 kDa, 700 - 750 kDa, and 750 - 800 kDa.**

Another preferred embodiment relates to the method of the first aspect, wherein the the second temperature is sufficiently higher than the first temperature to allow the *Bacillus* host cell to produce hyaluronic acid with a desired average molecular weight in a range selected from the group of molecular weight ranges consisting of **300 - 400 kDa, 400 - 500 kDa, 500 - 600 kDa, 600 - 700 kDa, 700 - 800 kDa.**

The level of hyaluronic acid produced by a Bacillus host cell of the present invention may be determined according to the modified carbazole method (Bitter and Muir, 1962, Anal Biochem. 4: 330-334). Moreover, the average molecular weight of the hyaluronic acid may be determined using standard methods in the art, such as those described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, California.

The hyaluronic acid obtained by the methods of the present invention may be subjected to various techniques known in the art to modify the hyaluronic acid, such as crosslinking as described, for example, in U.S. Patent Nos. 5,616,568, 5,652,347, and 5,874,417. Moreover, the molecular weight of the hyaluronic acid may be altered using techniques known in the art.

### Host Cells

In the methods of the present invention, the *Bacillus* host cell may be any *Bacillus* cell suitable for recombinant production of hyaluronic acid. The *Bacillus* host cell may be a wildtype *Bacillus* cell or a mutant thereof. *Bacillus* cells useful in the practice of the present invention include, but are not limited to, *Bacillus agaraderhens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. Mutant *Bacillus subtilis* cells particularly adapted for recombinant expression are described in WO 98/22598. Non-encapsulating *Bacillus* cells are particularly useful in the present invention.

In a preferred embodiment, the *Bacillus* host cell is a *Bacillus amyloliquefaciens, Bacillus clausii, Bacillus lentus, Bacillus licheniformis, Bacillus stearothermophilus* or *Bacillus subtilis* cell. In a more preferred embodiment, the *Bacillus* cell is a *Bacillus amyloliquefaciens* cell. In another more preferred embodiment, the *Bacillus* cell is a *Bacillus clausii* cell. In another more preferred embodiment, the *Bacillus* cell is a *Bacillus lentus* cell. In another more preferred embodiment, the *Bacillus* cell is a *Bacillus licheniformis* cell. In another more preferred embodiment, the *Bacillus* cell is a *Bacillus subtilis* cell. In a most preferred embodiment, the *Bacillus* host cell is *Bacillus subtilis A164A5* (see U.S.,Patent No. 5,891,701) or *Bacillus subtilis* 168Δ4.

Transformation of the *Bacillus* host cell with a nucleic acid construct of the present invention may, for instance, be effected by protoplast transformation (see, *e.g*., Chang and Cohen, 1979, Molecular General Genetics 168: 111-115), by using competent cells (see, *e.g*., Young and Spizizen, 1961, Journal of Bacteriology 81: 823-829, or Dubnau and Davidoff-Abelson, 1971, Journal of Molecular Biology 56: 209-221), by electroporation (see, *e.g*., Shigekawa and Dower, 1988, Biotechniques 6: 742-751), or by conjugation (see; *e.g.,* Koehler and Thorne, 1987, Journal of Bacteriology 169: 5271-5278).

### Nucleic Acid Constructs

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct may be synonymous with the term expression cassette when the nucleic acid construct contains all the control sequences required for expression of a coding sequence. The term "coding sequence" is defined herein as a sequence which is transcribed into mRNA and translated into an enzyme of interest when placed under the control of the below mentioned control sequences. The boundaries of the coding sequence are generally determined by a ribosome binding site located just upstream of the open reading frame at the 5' end of the mRNA and a transcription terminator sequence located just downstream of the open reading frame at the 3' end of the mRNA. A coding sequence can include, but is not limited to, DNA, cDNA, and recombinant nucleic acid sequences.

The techniques used to isolate or clone a nucleic acid sequence encoding a polypeptide are well known in the art and include, for example, isolation from genomic DNA, preparation from cDNA, or a combination thereof. The cloning of the nucleic acid sequences from such genomic DNA can be effected, *e.g*., by using antibody screening of expression libraries to detect cloned DNA fragments with shared structural features or the well known polymerase chain reaction (PCR). See, for example, Innis et al., 1990, PCR Protocols: A Guide to Methods and Application, Academic Press, New York. Other nucleic acid amplification procedures such as ligase chain reaction, ligated activated transcription, and nucleic acid sequence-based amplification may be used. The cloning procedures may involve excision and isolation of a desired nucleic acid fragment comprising the nucleic acid sequence encoding the polypeptide, insertion of the fragment into a vector molecule, and incorporation of the recombinant vector into a *Bacillus* cell where clones of the nucleic acid sequence will be replicated. The nucleic acid sequence may be of genomic, cDNA, RNA, semi-synthetic, synthetic origin, or any combinations thereof.

An isolated nucleic acid sequence encoding an enzyme may be manipulated in a variety of ways to provide for expression of the enzyme. Manipulation of the nucleic acid sequence prior to its insertion into a construct or vector may be desirable or necessary depending on the expression vector or *Bacillus* host cell. The techniques for modifying nucleic acid sequences utilizing cloning methods are well known in the art. It will be understood that the nucleic acid sequence may also be manipulated *in vivo* in the host cell using methods well known in the art.

A number of enzymes are involved in the biosynthesis of hyaluronic acid. These enzymes include hyaluronan synthase, UDP-glucose 6-dehydrogenase, UDP-glucose pyrophosphorylase, UDP-N-acetylglucosamine pyrophosphorylase, glucose-6-phosphate isomerase, hexokinase, phosphoglucomutase, amidotransferase, mutase, and acetyl transferase. Hyaluronan synthase is the key enzyme in the production of hyaluronic acid.

"Hyaluronan synthase" is defined herein as a synthase that catalyzes the elongation of a hyaluronan chain by the addition of GIcUA and GIcNAc sugar precursors. The amino acid sequences of streptococcal hyaluronan synthases, vertebrate hyaluronan synthases, and the viral hyaluronan synthase are distinct from the *Pasteurella* hyaluronan synthase, and have been proposed for classification as Group I and Group II hyaluronan synthases, the Group I hyaluronan synthases including Streptococcal hyaluronan synthases (DeAngelis, 1999). For production of hyaluronan in *Bacillus* host cells, hyaluronan synthases of a eukaryotic origin, such as mammalian hyaluronan synthases, are less preferred.

The hyaluronan synthase encoding sequence may be any nucleic acid sequence capable of being expressed in a *Bacillus* host cell. The nucleic acid sequence may be of any origin. Preferred hyaluronan synthase genes include any of either Group I or Group II, such as the Group I hyaluronan synthase genes from *Streptococcus equisimilis, Streptococcus pyogenes, Streptococcus uberis,* and *Streptococcus equi* subsp. *zooepidemicus,* or the Group II hyaluronan synthase genes of *Pasturella multocida.*

The methods of the present invention also include constructs whereby precursor sugars of hyaluronan are supplied to the host cell, either to the culture medium, or by being encoded by endogenous genes, by non-endogenous genes, or by a combination of endogenous and non-endogenous genes in the *Bacillus* host cell. The precursor sugar may be D-glucuronic acid or N-acetyl-glucosamine.

In the methods of the present invention, the nucleic acid construct may further comprise one or more genes encoding enzymes in the biosynthesis of a precursor sugar of a hyaluronan. Alternatively, the *Bacillus* host cell may further comprise one or more second nucleic acid constructs comprising one or more genes encoding enzymes in the biosynthesis of the precursor sugar. Hyaluronan production is improved by the use of constructs with a nucleic acid sequence or sequences encoding a gene or genes directing a step in the synthesis pathway of the precursor sugar of hyaluronan. By, "directing a step in the synthesis pathway of a precursor sugar of hyaluronan" is meant that the expressed protein of the gene is active in the formation of N-acetyl-glucosamine or D-glucuronic acid, or a sugar that is a precursor of either of N-acetyl-glucosamine and D-glucuronic acid.

In a preferred method for supplying precursor sugars, constructs are provided for improving hyaluronan production in a host cell having a hyaluronan synthase, by culturing a host cell having a recombinant construct with a heterologous promoter region operably linked to a nucleic acid sequence encoding a gene directing a step in the synthesis pathway of a precursor sugar of hyaluronan. In a preferred method the host cell also comprises a recombinant construct having a promoter region operably linked to a hyaluronan synthase, which may use the same or a different promoter region than the nucleic acid sequence to a synthase involved in the biosynthesis of N-acetyl-glucosamine. In a further preferred embodiment, the host cell may have a recombinant construct with a promoter region operably linked to different nucleic acid sequences encoding a second gene involved in the synthesis of a precursor sugar of hyaluronan.

Thus, the present disclosure also relates to constructs for improving hyaluronan production by the use of constructs with a nucleic acid sequence encoding a gene directing a step in the synthesis pathway of a precursor sugar of hyaluronan. The nucleic acid sequence to the precursor sugar may be expressed from the same or a different promoter as the nucleic acid sequence encoding the hyaluronan synthase.

The genes involved in the biosynthesis of precursor sugars for the production of hyaluronic acid include a UDP-glucose 6-dehydrogenase gene, UDP-glucose pyrophosphorylase gene, UDP-N-acetylglucosamine pyrophosphorylase gene, glucose-6-phosphate isomerase gene, hexokinase gene, phosphoglucomutase gene, amidotransferase gene, mutase gene, and acetyl transferase gene.

In a cell containing a hyaluronan synthase, any one or combination of two or more of *hasB, hasC* and *hasD,* or the homologs thereof, such as the *Bacillus subtilis tuaD, gtaB,* and *gcaD,* respectively, as well as *hasE,* may be expressed to increase the pools of precursor sugars available to the hyaluronan synthase. The *Bacillus* genome is described in Kunst, et al., Nature 390, 249-256, "The complete genome sequence of the Gram-positive bacterium Bacillus subtilis" (20 November 1997). In some instances, such as where the host cell does not have a native hyaluronan synthase activity, the construct may include the *hasA* gene.

The nucleic acid sequence encoding the biosynthetic enzymes may be native to the host cell, while in other cases heterologous sequence may be utilized. If two or more genes are expressed they may be genes that are associated with one another in a native operon, such as the genes of the HAS operon of *Streptococcus equisimilis,* which comprises hasA, *hasB, hasC* and *hasD.* In other instances, the use of some combination of the precursor gene sequences may be desired, without each element of the operon included. The use of some genes native to the host cell, and others which are exogenous may also be preferred in other cases. The choice will depend on the available pools of sugars in a given host cell, the ability of the cell to accommodate overproduction without interfering with other functions of the host cell, and whether the cell regulates expression from its native genes differently than exogenous genes.

As one example, depending on the metabolic requirements and growth conditions of the cell, and the available precursor sugar pools, it may be desirable to increase the production of N-acetyl-glucosamine by expression of a nucleic acid sequence encoding UDP-N-acetylglucosamine pyrophosphorylase, such as the *hasD* gene, the *Bacillus gcaD* gene, and homologs thereof. Alternatively, the precursor sugar may be D-glucuronic acid. In one such embodiment, the nucleic acid sequence encodes UDP-glucose 6-dehydrogenase. Such nucleic acid sequences include the *Bacillus tuaD* gene, the *hasB* gene of *Streptococcus,* and homologs thereof. The nucleic acid sequence, may also encode UDP-glucose pyrophosphorylase, such as in the *Bacillus gtaB* gene, the *hasC* gene of *Streptococcus,* and homologs thereof.

In the methods of the present invention, the UDP-glucose 6-dehydrogenase gene may be a *hasB* gene or *tuaD* gene; or homologs thereof.

In the methods of the present invention, the UDP-glucose pyrophosphorylase gene may be a *hasC* gene or *gtaB* gene; or homologs thereof.

In the methods of the present invention, the UDP-N-acetylglucosamine pyrophosphorylase gene may be a *hasD* or *gcaD* gene; or homologs thereof.

In the methods of the present invention, the glucose-6-phosphate isomerase gene may be a *hasE* or homolog thereof.

In the methods of the present invention, the hyaluronan synthase gene and the one or more genes encoding a precursor sugar are under the control of the same promoter. Alternatively, the one or more genes encoding a precursor sugar are under the control of the same promoter but a different promoter driving the hyaluronan synthase gene. A further alternative is that the hyaluronan synthase gene and each of the genes encoding a precursor sugar are under the control of different promoters. In a preferred embodiment, the hyaluronan synthase gene and the one or more genes encoding a precursor sugar are under the control of the same promoter.
In some cases the host cell will have a recombinant construct with a heterologous promoter region operably linked to a nucleic acid sequence encoding a gene directing a step in the synthesis pathway of a precursor sugar of hyaluronan, which may be in concert with the expression of hyaluronan synthase from a recombinant construct. The hyaluronan synthase may be expressed from the same or a different promoter region than the nucleic acid sequence encoding an enzyme involved in the biosynthesis of the precursor. In another preferred embodiment, the host cell may have a recombinant construct with a promoter region operably linked to a different nucleic acid sequence encoding a second gene involved in the synthesis of a precursor sugar of hyaluronan.

The nucleic acid sequence encoding the enzymes involved in the biosynthesis of the precursor sugar(s) may be expressed from the same or a different promoter as the nucleic acid sequence encoding the hyaluronan synthase. In the former sense, "artificial operons" are constructed, which may mimic the operon of *Streptococcus equisimilis* in having each *hasA, hasB, hasC and hasD,* or homologs thereof, or, alternatively, may utilize less than the full complement present in the *Streptococcus equisimilis* operon. The artificial operons" may also comprise a glucose-6-phosphate isomerase gene *(hasE)* as well as one or more genes selected from the group consisting of a hexokinase gene, phosphoglucomutase gene, amidotransferase gene, mutase gene, and acetyl transferase gene. In the artificial operon, at least one of the elements is heterologous to one other of the elements, such as the promoter region being heterologous to the encoding sequences.

In a preferred embodiment of the method, the nucleic acid construct comprises *hasA, tuaD,* and *gtaB.* In another preferred embodiment, the nucleic acid construct comprises *hasA, tuaD, gtaB,* and *gcaD.* In another preferred embodiment, the nucleic acid construct comprises *hasA* and *tuaD.* In another preferred embodiment, the nucleic acid construct comprises *hasA.* In another preferred embodiment, the nucleic acid construct comprises *hasA, tuaD, gtaB, gcaD,* and *hasE.* In another preferred embodiment, the nucleic acid construct comprises *hasA, hasB, hasC,* and *hasD.* In another preferred embodiment, the nucleic acid construct comprises *hasA, hasB, hasC, hasD,* and *hasE.* Based on the above preferred embodiments, the genes noted can be replaced with homologs thereof.

In the methods of the present invention, the nucleic acid constructs comprise a hyaluronan synthase encoding sequence operably linked to a promoter sequence foreign to the hyaluronan synthase encoding sequence. The promoter sequence may be, for example, a single promoter or a tandem promoter.

"Promoter" is defined herein as a nucleic acid sequence involved in the binding of RNA polymerase to initiate transcription of a gene. "Tandem promoter" is defined herein as two or more promoter sequences each of which is operably linked to a coding sequence and mediates the transcription of the coding sequence into mRNA. "Operably linked" is defined herein as a configuration in which a control sequence, *e.g*., a promoter sequence, is appropriately placed at a position relative to a coding sequence such that the control sequence directs the production of a polypeptide encoded by the coding sequence. As noted earlier, a "coding sequence" is defined herein as a nucleic acid sequence which is transcribed into mRNA and translated into a polypeptide when placed under the control of the appropriate control sequences. The boundaries of the coding sequence are generally determined by a ribosome binding site located just upstream of the open reading frame at the 5' end of the mRNA and a transcription terminator sequence located just downstream of the open reading frame at the 3' end of the mRNA. A coding sequence can include, but is not limited to, genomic DNA, cDNA, semisynthetic, synthetic, and recombinant nucleic acid sequences.

In a preferred embodiment of the method, the promoter sequences may be obtained from a bacterial source. In a more preferred embodiment, the promoter sequences may be obtained from a gram positive bacterium such as a *Bacillus* strain, *e.g., Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis;* or a *Streptomyces* strain, *e.g., Streptomyces lividans* or *Streptomyces murinus;* or from a gram negative bacterium, *e.g., E. coli* or *Pseudomonas* sp.

Examples of suitable promoters for directing the transcription of a nucleic acid sequence in the methods of the present invention are the promoters obtained from the *E. coli lac* operon, *Streptomyces coelicolor* agarase gene *(dagA), Bacillus lentus or Bacillus clausii* alkaline protease gene (*aprH*), *Bacillus licheniformis* alkaline protease gene (subtilisin Carlsberg gene), *Bacillus subtilis* levansucrase gene *(sacB), Bacillus* subtilis alpha-amylase gene (*amyE*), *Bacillus licheniformis* alpha-amylase gene *(amyL), Bacillus stearothermophilus* maltogenic amylase gene (*amyM*), *Bacillus amyloliquefaciens* alpha-amylase gene (*amyQ*), *Bacillus licheniformis* penicillinase gene *(penP), Bacillus subtilis xylA* and *xylB* genes, *Bacillus thuringiensis* subsp. *tenebrionis* CrylllA gene *(cryIIIA)* or portions thereof, prokaryotic beta-lactamase gene (Villa-Kamaroff et al., 1978, Proceedings of the National Academy of Sciences USA 75:3727-3731). Other examples are the promoter of the spo1 bacterial phage promoter and the tac promoter (DeBoer et al., 1983, Proceedings of the National Academy of Sciences USA 80:21-25). Further promoters are described in "Useful proteins from recombinant bacteria" in Scientific American, 1980, 242:74-94; and in Sambrook, Fritsch, and Maniatus, 1989, Molecular Cloning, A Laboratory Manual, 2d edition, Cold Spring Harbor, New York.

The promoter may also be a "consensus" promoter having the sequence TTGACA for the "-35" region and TATAAT for the "-10" region. The consensus promoter may be obtained from any promoter which can function in a *Bacillus* host cell. The construction of a "consensus" promoter may be accomplished by site-directed mutagenesis to create a promoter which conforms more perfectly to the established consensus sequences for the "-10" and "-35" regions of the vegetative "sigma A-type" promoters for *Bacillus subtilis* (Voskuil et al., 1995, Molecular Microbiology 17: 271-279).

In a preferred embodiment, the "consensus" promoter is obtained from a promoter obtained from the *E*. *coli lac* operon, *Streptomyces coelicolor* agarase gene *(dagA), Bacillus clausii* or *Bacillus lentus* alkaline protease gene *(aprH)*, *Bacillus licheniformis* alkaline protease gene (subtilisin Carlsberg gene), *Bacillus subtilis* levansucrase gene *(sacB), Bacillus* subtilis alpha-amylase gene *(amyE), Bacillus licheniformis* alpha-amylase gene *(amyL), Bacillus stearothermophilus* maltogenic amylase gene *(amyM), Bacillus amyloliquefaciens* alpha-amylase gene *(amyQ), Bacillus licheniformis* penicillinase gene *(penP), Bacillus subtilis xylA* and *xylB* genes, *Bacillus thuringiensis* subsp. *tenebrionis* CrylllA gene *(cryIIIA)* or portions thereof, or prokaryotic beta-lactamase gene *spo1* bacterial phage promoter. In a more preferred embodiment, the "consensus" promoter is obtained from *Bacillus amyloliquefaciens* alpha-amylase gene *(amyQ).*

Widner, *et al.,* United States Patent Nos. 6,255,076 and 5,955,310, describe tandem promoters and constructs and methods for use in expression in *Bacillus* cells, including the short consensus *amyQ* promoter (also called scBAN). The use of the *cryIIIA* stabilizer sequence, and constructs using the sequence, for improved production in *Bacillus* are also described therein.

Each promoter sequence of the tandem promoter may be any nucleic acid sequence which shows transcriptional activity in the *Bacillus* cell of choice including a mutant, truncated, and hybrid promoter, and may be obtained from genes encoding extracellular or intracellular polypeptides either homologous or heterologous to the *Bacillus* cell. Each promoter sequence may be native or foreign to the nucleic acid sequence encoding the polypeptide and native or foreign to the *Bacillus* cell. The promoter sequences may be the same promoter sequence or different promoter sequences.

The two or more promoter sequences of the tandem promoter may simultaneously promote the transcription of the nucleic acid sequence. Alternatively, one or more of the promoter sequences of the tandem promoter may promote the transcription of the nucleic acid sequence at different stages of growth of the *Bacillus* cell.

In a preferred embodiment, the tandem promoter contains at least the *amyQ* promoter of the *Bacillus amyloliquefaciens* alpha-amylase gene. In another preferred embodiment, the tandem promoter contains at least a "consensus" promoter having the sequence TTGACA for the "-35" region and TATAAT for the "-10" region. In another preferred embodiment, the tandem promoter contains at least the *amyL* promoter of the *Bacillus licheniformis* alpha-amylase gene. In another preferred embodiment, the tandem promoter contains at least the *cryIIIA* promoter or portions thereof (Agaisse and Lereclus, 1994, Molecular Microbiology 13: 97-107).

In a more preferred embodiment, the tandem promoter contains at least the *amyL* promoter and the *cryIIIA* promoter. In another more preferred embodiment, the tandem promoter contains at least the *amyQ* promoter and the *cryIIIA* promoter. In another more preferred embodiment, the tandem promoter contains at least a "consensus" promoter having the sequence TTGACA for the "-35" region and TATAAT for the "-10" region and the *cryIIIA* promoter. In another more preferred embodiment, the tandem promoter contains at least two copies of the *amyL* promoter. In another more preferred embodiment, the tandem promoter contains at least two copies of the *amyQ* promoter. In another more preferred embodiment, the tandem promoter contains at least two copies of a "consensus" promoter having the sequence TTGACA for the "-35" region and TATAAT for the "-10" region. In another more preferred embodiment, the tandem promoter contains at least two copies of the *cryIIIA* promoter.

"An mRNA processing/stabilizing sequence" is defined herein as a sequence located downstream of one or more promoter sequences and upstream of a coding sequence to which each of the one or more promoter sequences are operably linked such that all mRNAs synthesized from each promoter sequence may be processed to generate mRNA transcripts with a stabilizer sequence at the 5' end of the transcripts. The presence of such a stabilizer sequence at the 5' end of the mRNA transcripts increases their half-life (Agaisse and Lereclus, 1994, *supra,* Hue et a/., 1995, Journal of Bacteriology 177: 3465-3471). The mRNA processing/stabilizing sequence is complementary to the 3' extremity of a bacterial 16S ribosomal RNA. In a preferred embodiment of the method, the mRNA processing/stabilizing sequence generates essentially single-size transcripts with a stabilizing sequence at the 5' end of the transcripts. The mRNA processing/stabilizing sequence is preferably one, which is complementary to the 3' extremity of a bacterial 16S ribosomal RNA. See, U.S. Patent Nos. 6,255,076 and 5,955,310.

In a more preferred embodiment, the mRNA processing/stabilizing sequence is the *Bacillus thuringiensis cryIIIA* mRNA processing/stabilizing sequence disclosed in WO 94/25612 and Agaisse and Lereclus, 1994, *supra,* or portions thereof which retain the mRNA processing/stabilizing function. In another more preferred embodiment, the mRNA processing/stabilizing sequence is the *Bacillus subtilis* SP82 mRNA processing/stabilizing sequence disclosed in Hue *et al.,* 1995, *supra,* or portions thereof which retain the mRNA processing/stabilizing function.

When the *cryIIIA* promoter and its mRNA processing/stabilizing sequence are employed in the methods of the present invention, a DNA fragment containing the sequence disclosed in WO 94/25612 and Agaisse and Lereclus, 1994, *supra,* or portions thereof which retain the promoter and mRNA processing/stabilizing functions, may be used. Furthermore, DNA fragments containing only the *cryIIIA* promoter or only the *cryIIIA* mRNA processing/stabilizing sequence may be prepared using methods well known in the art to construct various tandem promoter and mRNA processing/stabilizing sequence combinations. In this embodiment, the *cryIIIA* promoter and its mRNA processing/stabilizing sequence are preferably placed downstream of the other promoter sequence(s) constituting the tandem promoter and upstream of the coding sequence of the gene of interest.

The isolated nucleic acid sequence encoding the desired enzyme(s) involved in hyaluronic acid production may then be further manipulated to improve expression of the nucleic acid sequence. Expression will be understood to include any step involved in the production of the polypeptide including, but not limited to, transcription, post-transcriptional modification, translation, post-translational modification, and secretion. The techniques for modifying nucleic acid sequences utilizing cloning methods are well known in the art.

A nucleic acid construct comprising a nucleic acid sequence encoding an enzyme may be operably linked to one or more control sequences capable of directing the expression of the coding sequence in a *Bacillus* cell under conditions compatible with the control sequences.

The term "control sequences" is defined herein to include all components which are necessary or advantageous for expression of the coding sequence of a nucleic acid sequence. Each control sequence may be native or foreign to the nucleic acid sequence encoding the enzyme. In addition to promoter sequences described above, such control sequences include, but are not limited to, a leader, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding an enzyme.

The control sequence may also be a suitable transcription terminator sequence, a sequence recognized by a *Bacillus* cell to terminate transcription. The terminator sequence is operably linked to the 3' terminus of the nucleic acid sequence encoding the enzyme or the last enzyme of an operon. Any terminator which is functional in the *Bacillus* cell of choice may be used in the present invention.

The control sequence may also be a suitable leader sequence, a nontranslated region of a mRNA which is important for translation by the *Bacillus* cell. The leader sequence is operably linked to the 5' terminus of the nucleic acid sequence encoding the enzyme. Any leader sequence which is functional in the *Bacillus* cell of choice may be used in the present invention.

The control sequence may also be a signal peptide coding region, which codes for an amino acid sequence linked to the amino terminus of a polypeptide which can direct the expressed polypeptide into the cell's secretory pathway. The signal peptide coding region may be native to the polypeptide or may be obtained from foreign sources. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to that portion of the coding sequence which encodes the secreted polypeptide. The foreign signal peptide coding region may be required where the coding sequence does not normally contain a signal peptide coding region. Alternatively, the foreign signal peptide coding region may simply replace the natural signal peptide coding region in order to obtain enhanced secretion of the polypeptide relative to the natural signal peptide coding region normally associated with the coding sequence. The signal peptide coding region may be obtained from an amylase or a protease gene from a *Bacillus* species. However, any signal peptide coding region capable of directing the expressed polypeptide into the secretory pathway of a *Bacillus* cell of choice may be used in the present invention.

An effective signal peptide coding region for *Bacillus* cells is the signal peptide coding region obtained from the maltogenic amylase gene from *Bacillus* NCIB 11837, the *Bacillus stearothermophilus* alpha-amylase gene, the *Bacillus licheniformis* subtilisin gene, the *Bacillus licheniformis* beta-lactamase gene, the *Bacillus stearothermophilus* neutral proteases genes *(nprT, nprS, nprM),* and the *Bacillus subtilis prsA* gene. Further signal peptides are described by Simonen and Palva, 1993, Microbiological Reviews 57:109-137.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis alkaline* protease *(aprE)* and *Bacillus subtilis* neutral protease *(nprT).*

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac*, *tac,* and *trp* operator systems.

### Expression Vectors

In the methods of the present invention, a recombinant expression vector comprising a nucleic acid sequence, a promoter, and transcriptional and translational stop signals may be used for the recombinant production of an enzyme involved in hyaluronic acid production. The various nucleic acid and control sequences described above may be joined together to produce a recombinant expression vector which may include one or more convenient restriction sites to allow for insertion or substitution of the nucleic acid sequence encoding the polypeptide or enzyme at such sites. Alternatively, the nucleic acid sequence may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector which can be conveniently subjected to recombinant DNA procedures and can bring about the expression of the nucleic acid sequence. The choice of the vector will typically depend on the compatibility of the vector with the *Bacillus* cell into which the vector is to be introduced. The vectors may be linear or closed circular plasmids. The vector may be an autonomously replicating vector, i.e., a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication. Alternatively, the vector may be one which, when introduced into the *Bacillus* cell, is integrated into the genome and replicated together with the chromosome(s) into which it has been integrated. The vector system may be a single vector or plasmid or two or more vectors or plasmids which together contain the total DNA to be introduced into the genome of the *Bacillus* cell, or a transposon may be used.

The vectors preferably contain an element(s) that permits integration of the vector into the *Bacillus* host cell's genome or autonomous replication of the vector in the cell independent of the genome.

For integration into the host cell genome, the vector may rely on the nucleic acid sequence encoding the polypeptide or any other element of the vector for integration of the vector into the genome by homologous or nonhomologous recombination. Alternatively, the vector may contain additional nucleic acid sequences for directing integration by homologous recombination into the genome of the *Bacillus* cell. The additional nucleic acid sequences enable the vector to be integrated into the *Bacillus* cell genome at a precise location in the chromosome. To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the *Bacillus* cell. Furthermore, the integrational elements may be non-encoding or encoding nucleic acid sequences. On the other hand, the vector may be integrated into the genome of the host cell by non-homologous recombination.

For autonomous replication, the vector may further comprise an origin of replication enabling the vector to replicate autonomously in the *Bacillus* cell in question. Examples of bacterial origins of replication are the origins of replication of plasmids pUB110, pE194, pTA1060, and pAMB1 permitting replication in *Bacillus.* The origin of replication may be one having a mutation to make its function temperature-sensitive in the *Bacillus* cell (see, *e.g.,* Ehrlich, 1978, Proceedings of the National Academy of Sciences USA 75:1433).

The vectors preferably contain one or more selectable markers which permit easy selection of transformed cells. A selectable marker is a gene the product of which provides for biocide resistance, resistance to heavy metals, prototrophy to auxotrophs, and the like. Examples of bacterial selectable markers are the *dal* genes from *Bacillus subtilis* or *Bacillus licheniformis,* or markers which confer antibiotic resistance such as ampicillin, kanamycin, chloramphenicol or tetracycline resistance. Furthermore, selection may be accomplished by co-transformation, *e.g.,* as described in WO 91/09129, where the selectable marker is on a separate vector.

More than one copy of a nucleic acid sequence may be inserted into the host cell to increase production of the gene product. An increase in the copy number of the nucleic acid sequence can be obtained by integrating at least one additional copy of the sequence into the host cell genome or by including an amplifiable selectable marker gene with the nucleic acid sequence where cells containing amplified copies of the selectable marker gene, and thereby additional copies of the nucleic acid sequence, can be selected for by cultivating the cells in the presence of the appropriate selectable agent. A convenient method for achieving amplification of genomic DNA sequences is described in WO 94/14968.

The procedures used to ligate the elements described above to construct the recombinant expression vectors are well known to one skilled in the art (see, *e.g*., Sambrook *et al.,* 1989, supra).

### Production

In the methods of the present invention, the *Bacillus* host cells are cultivated in a nutrient medium suitable for production of the hyaluronic acid using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the enzymes involved in hyaluronic acid synthesis to be expressed and the hyaluronic acid to be isolated. The cultivation takes place in a suitable nutrient medium comprising carbon and nitrogen sources and inorganic salts, using procedures known in the art. Suitable media are available from commercial suppliers or may be prepared according to published compositions (*e.g*., in catalogues of the American Type Culture Collection). The secreted hyaluronic acid can be recovered directly from the medium.

The resulting hyaluronic acid may be isolated by methods known in the art. For example, the hyaluronic acid may be isolated from the nutrient medium by conventional procedures including, but not limited to, centrifugation, filtration, extraction, spray-drying, evaporation, or precipitation. The isolated hyaluronic acid may then be further purified by a variety of procedures known in the art including, but not limited to, chromatography (*e.g*., ion exchange, affinity, hydrophobic, chromatofocusing, and size exclusion), electrophoretic procedures (*e.g*., preparative isoelectric focusing), differential solubility (*e.g*., ammonium sulfate precipitation), or extraction (see, *e.g.,* Protein Purification, J.-C. Janson and Lars Ryden, editors, VCH Publishers, New York, 1989).

In the methods of the present invention, the *Bacillus* host cells produce greater than about 4 g, preferably greater than about 6 g, more preferably greater than about 8 g, even more preferably greater than about 10 g, and most preferably greater than about 12 g of hyaluronic acid per liter.

### Dotations/Disruptions

Gene deletion or replacement techniques may be used for the complete removal of a selectable marker gene or other undesirable gene. In such methods, the deletion of the selectable marker gene may be accomplished by homologous recombination using a plasmid that has been constructed to contiguously contain the 5' and 3' regions flanking the selectable marker gene. The contiguous 5' and 3' regions may be introduced into a *Bacillus* cell on a temperature-sensitive plasmid, *e.g.*, pE194, in association with a second selectable marker at a permissive temperature to allow the plasmid to become established in the cell. The cell is then shifted to a non-permissive temperature to select for cells that have the plasmid integrated into the chromosome at one of the homologous flanking regions. Selection for integration of the plasmid is effected by selection for the second selectable marker. After integration, a recombination event at the second homologous flanking region is stimulated by shifting the cells to the permissive temperature for several generations without selection. The cells are plated to obtain single colonies and the colonies are examined for loss of both selectable markers (see, for example, Perego, 1993, In A.L. Sonneshein, J.A. Hoch, and R. Losick, editors, Bacillus subtilis and Other Gram-Positive Bacteria, Chapter 42, American Society of Microbiology, Washington, D.C., 1993).

A selectable marker gene may also be removed by homologous recombination by introducing into the mutant cell a nucleic acid fragment comprising 5' and 3' regions of the defective gene, but lacking the selectable marker gene, followed by selecting on the counter-selection medium. By homologous recombination, the defective gene containing the selectable marker gene is replaced with the nucleic acid fragment lacking the selectable marker gene. Other methods known in the art may also be used.

U.S. Patent No. 5,891,701 discloses techniques for deleting several genes including *spollAC, aprE, nprE,* and *amyE.*

Other undesirable biological compounds may also be removed by the above described methods such as the red pigment synthesized by *cypX* (accession no. BG12580) and/or *yvmC* (accession no. BG14121).

In a preferred embodiment, the *Bacillus* host cell is unmarked with any heterologous or exogenous selectable markers. In another preferred embodiment, the *Bacillus* host cell does not produce any red pigment synthesized by *cypX* and *yvmC.*

### EXAMPLES

### Example 1

A recombinant *Bacillus* strain was constructed as disclosed in detail in WO 2003054163. This strain was then cultivated as follows: First a seed stage on agar at a constant temperature, then a seed stage in a stirred tank at constant temperature, and finally a fed batch main fermentation in a stirred tank at an initial temperature favourable for growth of the *Bacillus* strain, *e.g.* 37°C. Later, after the initiation, the fermentation temperature was shifted up or down in separate experiments to various other set temperatures in the range of 17 - 52°C. The temperature was then kept constant over a period of 7h, following the initiation of the fed batch phase.

The *Bacillus* strain was fermented in standard small fermenters in a medium composed per liter of 6.5 g of KH2PO4, 4.5 g of Na2HPO4, 3.0 g of (NH4)2SO4, 2.0 g of Na3-citrate-2H2O, 3.0 g of MgSO4,7H2O, 6.0 ml of Mikrosoy-2, 0.15 mg of biotin (1 ml of 0.15 mg/ml ethanol), 15.0 g of sucrose, 1.0 ml of SB 2066, 2.0 ml of P2000, 0.5 g of CaCl2·2H2O. The medium was pH 6.3 to 6.4 (unadjusted) prior to autoclaving. The CaCl2·2H2O was added after autoclaving.

The seed medium used was B-3, i.e., Agar-3 without agar, or "S/S-1" medium. The Agar-3 medium was composed per liter of 4.0 g of nutrient broth, 7.5 g of hydrolyzed protein, 3.0 g of yeast extract, 1.0 g of glucose, and 2% agar. The pH was not adjusted; pH before autoclaving was approximately 6.8; after autoclaving approximately pH 7.7.

The sucrose/soy seed flask medium (S/S-1) was composed per liter of 65 g of sucrose, 35 g of soy flour, 2 g of Na3-citrate-2H2O, 4 g of KH2P04, 5 g of Na2HPO4, and 6 ml of trace elements. The medium was adjusted pH to about 7 with NaOH; after dispensing the medium to flasks, 0.2% vegetable oil was added to suppress foaming. Trace elements was composed per liter of 100 g of citric acid-H2O, 20 g of FeSO4·7H2O, 5 g of MnSO4·H2O, 2 g of CuSO4-5H2O, and 2 g of ZnCl2.

The pH was adjusted to 6.8 - 7.0 with ammonia before inoculation, and controlled thereafter at pH 7.0 + 0.2 with ammonia and H3PO4. The temperature was maintained at 37°C. Agitation was at a maximum of 1300 RPM using two 6-bladed rushton impellers of 6 cm diameter in 3 liter tank with initial volume of 1.5 liters. The aeration had a maximum of 1.5 WM.

For feed, a simple sucrose solution was used. Feed started at about 4 hours after inoculation, when dissolved oxygen (D.O.) was still being driven down (i.e., before sucrose depletion). The temperature was shifted to a pre-selected higher temperature in the range of 37 - 52°C. The feed rate was then ramped linearly from 0 to approximately 6 g sucrose/L0-hr over the 7 hour time span. A lower feed rate, ramped linearly from 0 to approximately 2 g sucrose/L0-hr, was also used in some fermentations.

Cells were removed by diluting 1 part culture with 3 parts water, mixing well and centrifuging at about 30,000 x g to produce a clear supernatant and cell pellet, which can be washed and dried.

Assays of hyaluronic acid concentration were performed using the ELISA method, based on a hyaluronan binding protein (protein and kits commercially available from Seikagaku America, Falmouth, MA). Hyaluronic acid concentrations were determined using the modified carbazole method (Bitter and Muir, 1962, Anal Biochem. 4: 330-334).

Molecular weights were determined using a GPC MALLS assay. Data was gathered from GPC MALLS assays using the following procedure. GPC-MALLS (gel permeation or size-exclusion) chromatography coupled with multi-angle laser light scattering) is widely used to characterize high molecular weight (MW) polymers. Separation of polymers is achieved by GPC, based on the differential partitioning of molecules of different MW between eluent and resin. The average molecular weight of an individual polymer is determined by MALLS based the differential scattering extent/angle of molecules of different MW. Principles of GPC-MALLS and protocols suited for hyaluronic acid are described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, California). An Agilent 1100 isocratic HPLC, a Tosoh Biosep G6000 PWxI column for the GPC, and a Wyatt Down EOS for the MALLS were used. An Agilent G1362A refractive index detector was linked downstream from the MALLS for eluate concentration determination. Various commercial hyaluronic acid products with known molecular weights served as standards.

The results are shown in figure 1 as the trends for the average molecular weight at the end of fermentations as a function of the final fermentation temperature, which had been kept constant for 7 hours, as determined by GPC-MALLS. The figure surprisingly shows that a desired MW can be selected through careful selection and manipulation of the fermentation temperatures. There is a maximum MW at low final temperatures of 17°C, and a minimum MW at high final fermentation temperatures of 52°C.

## Claims

1. A method for producing a hyaluronic acid with a low average molecular weight by temperature-controlled fermentation, the method comprising the steps of:
(a) cultivating a recombinant *Bacillus* host cell at a first temperature conducive to its growth, wherein the *Bacillus* host cell comprises a nucleic acid construct comprising a hyaluronan synthase encoding sequence operably linked to a promoter sequence foreign to the hyaluronan synthase encoding sequence;
(b) then cultivating the recombinant *Bacillus* host cell of step (a) at a second temperature higher than the first temperature of step (a) under conditions suitable for production of the hyaluronic acid, where the second temperature is in the range 40°C - 52°C and at least 5°C higher than the first temperature, whereby the *Bacillus* host cell produces hyaluronic acid with a desired average molecular weight in the range selected from the group of molecular weight ranges consisting of 300 - 400 kDa, 400 - 500 kDa, 500 - 600 kDa, 600 - 700 kDa, 700 - 800 kDa; and
(c) recovering the hyaluronic acid.

2. The method of claim 1, wherein the hyaluronan synthase encoding sequence encodes a Group I hyaluronan synthase.

3. The method of claim 1, wherein the hyaluronan synthase encoding sequence encodes a Group II hyaluronan synthase.

4. The method of any of claims 1-3, wherein a precursor sugar of the hyaluronic acid is supplied to or produced by the *Bacillus* host cell.

5. The method of claim 4, wherein the precursor sugar is encoded by endogenous genes, by non-endogenous genes, or by a combination of endogenous and non-endogenous genes in the *Bacillus* host cell.

6. The method of any of claims 1-5, wherein the nucleic acid construct further comprises one or more genes encoding enzymes in the biosynthesis of a precursor sugar of the hyaluronic acid or the *Bacillus* host cell further comprises one or more second nucleic acid constructs comprising one or more genes encoding enzymes in the biosynthesis of a precursor sugar of the hyaluronic acid.

7. The method of claim 6, wherein the one or more genes is selected from the group consisting of a UDP-glucose 6-dehydrogenase gene, UDP-glucose pyrophosphorylase gene, UDP-N-acetylglucosamine pyrophosphorylase gene, glucose-6-phosphate isomerase gene, hexokinase gene, phosphoglucomutase gene, amidotransferase gene, mutase gene, and acetyl transferase gene.

8. The method of claim 7, wherein the UDP-glucose 6-dehydrogenase gene is a *hasB* gene or *tuaD* gene; or homologs thereof.

9. The method of claim 7, wherein the UDP-glucose pyrophosphorylase gene is a *hasC* gene or *gtaB* gene; or homologs thereof.

10. The method of claim 7, wherein the UDP-N-acetylglucosamine pyrophosphorylase gene is a *hasD* or *gcaD* gene; or homologs thereof.

11. The method of claim 7, wherein the glucose-6-phosphate isomerase gene is a *hasE* gene or homologue thereof.

12. The method of any of claims 6-11, wherein the one or more genes encoding a precursor sugar is under the control of the same or a different promoter(s) as the hyaluronan synthase encoding sequence.

13. The method of claim 12, wherein the same or the different promoter sequence(s) is a tandem promoter in which each promoter of the tandem promoter is operably linked to the hyaluronan synthase encoding sequence.

14. The method of any of claims 1-13, wherein the nucleic acid construct further comprises an mRNA processing/stabilizing sequence located downstream of the promoter sequence and upstream of the hyaluronan synthase encoding sequence.

15. The method of claim 14, wherein the nucleic acid construct further comprises an mRNA processing/stabilizing sequence located downstream of a different promoter or different promoters of the one or more genes encoding enzymes in the biosynthesis of the precursor sugar and upstream of the one or more genes.

16. The method of any of claims 1-15, wherein the *Bacillus* host cell is selected from the group consisting of *Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis.*

17. The method of any of claims 1-16, wherein the nucleic acid construct comprises a hyaluronan synthase gene, UDP-glucose 6-dehydrogenase gene, and UDP-glucose pyrophosphorylase gene operably linked to a short "consensus" promoter of *amyQ* having the sequence TTGACA for the "-35" region and TATAAT for the "-10" region.

18. The method of any of claims 1-17, wherein the *Bacillus* host cell contains a disrupted or deleted *cypX* and/or yvmC gene.

19. The method of any of claims 1-18, wherein the first temperature is in the range of 30°C-40°C.

20. The method of any of claims 1-19, wherein the cultivating step at the second temperature takes up at least 20% of the total cultivating time.

21. The method of any of claims 1-20, wherein the second temperature is sufficiently higher than the first temperature to allow the Bacillus host cell to produce hyaluronic acid with a desired average molecular weight in a range selected from the group of molecular weight ranges consisting of 300 - 350 kDa, 350 - 400 kDa, 400 - 450 kDa, 450 - 500 kDa, 500 - 550 kDa, 550 - 600 kDa, 600 - 650 kDa, 650 - 700 kDa, 700 - 750 kDa, and 750 - 800 kDa.

## Patentansprüche

1. Verfahren zur Herstellung einer Hyaluronsäure mit einem niederen Molekulargewicht durch temperaturkontrollierte Fermentation, wobei das Verfahren die folgenden Schritte umfasst:
(a) Kultivieren einer rekombinanten *Bacillus-Wirtszelle* bei einer ersten, ihrem Wachstum zuträglichen Temperatur, wobei die *Bacillus-Wirtszelle* ein Nukleinsäurekonstrukt einschließt, das eine Hyaluronansynthase-kodierende Sequenz umfasst, die mit einer Promotorsequenz, die zu der Hyaluronansynthase-kodierende Sequenz fremd ist, operabel verknüpft ist;
(b) anschließend Kultivieren der rekombinaten Bacillus-Wirtszelle von Schritt (a) bei einer zweiten Temperatur, die höher ist als die erste Temperatur von Schritt (a) unter Bedingungen, die zur Herstellung der Hyaluronsäure geeignet sind, wobei die zweite Temperatur in Bereich von 40 °C bis 52 °C liegt und mindestens 5 °C höher ist als die erste Temperatur, wobei die *Bacillus-*Wirtszelle Hyaluronsäure mit einem gewünschten mittleren Molekulargewicht in dem Bereich, ausgewählt aus der Gruppe der Molekulargewichtsbereiche bestehend aus 300 - 400 kDa, 400 - 500 kDa, 500 - 600 kDa, 600 - 700 kDa, 700-800 kDa, produziert; und
(c) Gewinnen der Hyaluronsäure.

2. Verfahren nach Anspruch 1, wobei die Hyaluronansynthase-kodierende Sequenz, eine Gruppe I-Hyaluronansynthase kodiert.

3. Verfahren nach Anspruch 1, wobei die Hyaluronansynthase-kodierende Sequenz, eine Gruppe II-Hyaluronansynthase kodiert.

4. Verfahren nach einem der Ansprüche 1-3, wobei ein Vorläufer-Zucker der Hyaluronsäure für die *Bacillus-Wirtszelle* bereitgestellt wird oder von ihr produziert wird.

5. Verfahren nach Anspruch 4, wobei der Vorläufer-Zucker durch endogene Gene, durch nicht endogene Gene oder durch eine Kombination von endogenen und nicht endogenen Genen in der *Bacillus-Wirtszelle* kodiert wird.

6. Verfahren nach einem der Ansprüche 1-5, wobei das Nucleinsäurekonstrukt weiterhin ein oder mehrere Gene umfasst, die bei der Biosynthese eines Vorläufer-Zuckers der Hyaluronsäure Enzyme kodieren, oder die *Bacillus-Wirtszelle* weiterhin ein oder mehrere zweite Nukeleinsäurekonstrukte umfasst, die ein oder mehrere Gene umfassen, die bei der Biosynthese eines Vorläufer-Zuckers der Hyaluronsäure Enzyme kodieren.

7. Verfahren nach Anspruch 6, wobei das eine oder die mehreren Gene aus der Gruppe ausgewählt sind bestehend aus einem UDP-Glucose-6-dehydrogenasegen, UDP-Glucosepyrophosphorylasegen, UDP-N-Acetylglucosaminpyrophosphorylasegen, Glucose-6-phosphatisomerasegen, Hexokinasegen, Phosphoglucomutasegen, Amidotransferasegen, Mutasegen, und einem Acetyltransferasegen.

8. Verfahren nach Anspruch 7, wobei das UDP-Glucose-6-dehydrogenasegen ein *has*B-Gen oder ein *tuaD-Gen* ist; oder Homologe davon.

9. Verfahren nach Anspruch 7, wobei das UDP-Glucosepyrophosphorylasgen ein *has*C-Gen oder ein *gta*B-Gen ist; oder Homologe davon.

10. Verfahren nach Anspruch 7, wobei das UDP-N-Acetylglucosaminpyrophosphorylasegen ein *hasD-*Gen oder ein *gcaD-Gen* ist; oder Homologe davon.

11. Verfahren nach Anspruch 7, wobei das Glucose-6-phosphatisomerasegen ein *hasE-*Gen oder Homolog davon ist.

12. Verfahren nach einem der Ansprüche 6-11, wobei das eine oder die mehreren Gene, die einen Vorläufer-Zucker kodieren, unter der Kontrolle des gleichen oder eines verschiedenen Promotors(en) stehen wie die Hyaluronansynthase-kodierende Sequenz.

13. Verfahren nach Anspruch 12, wobei die gleiche oder die verschiedenen Promotorsequenz(en) ein Tandem-Promotor sind, in dem jeder Promotor des Tandem-Promotors operabel mit der Hyaluronansynthase-kodierenden Sequenz verknüpft ist.

14. Verfahren nach einem der Ansprüche 1-13, wobei das Nucleinsäurekonstrukt weiterhin eine mRNA-prozessierende/stabilisierende Sequenz umfasst, die stromabwärts zu der Promotorsequenz und stromaufwärts zu der Hyaluronansynthase-kodierenden Sequenz lokalisiert ist.

15. Verfahren nach Anspruch 14, wobei das Nucleinsäurekonstrukt weiterhin eine mRNA-prozessierende/stabilisierende Sequenz umfasst, die stromabwärts zu einem verschiedenen Promotor oder verschiedenen Promotoren des einen oder der mehreren Gene, die bei der Biosynthese des Vorläufer-Zuckers Enzyme kodieren, und stromaufwärts zu dem einen oder den mehreren Genen lokalisiert ist.

16. Verfahren nach einem der Ansprüche 1-15, wobei die *Bacillus*-Wirtszelle aus der Gruppe ausgewählt ist, bestehend aus *Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* und *Bacillus thuringiensis.*

17. Verfahren nach einem der Ansprüche 1-16, wobei das Nucleinsäurekonstrukt ein Hyaluronansynthasegen, ein UDP-Glucose-6-dehydrogenasegen, und ein UDP-Glucosepyrophosphorylasegen umfasst, das mit einem kurzen "consensus" Promotor von *amy*Q mit der Sequenz TTGACA für die "-35"-Region und TATAAT für die "-10"-Region operabel verknüpft ist.

18. Verfahren nach einem der Ansprüche 1-17, wobei die *Bacillus*-Wirtszelle ein unterbrochenes oder deletiertes *cyp*X-Gen und/oder *yvm*C-Gen enthält.

19. Verfahren nach einem der Ansprüche 1-18, wobei die erste Temperatur im Bereich von 30 °C - 40 °C liegt.

20. Verfahren nach einem der Ansprüche 1-19, wobei der Kultivierungsschritt bei der zweiten Temperatur mindestens 20 % der gesamten Kultivierungszeit beansprucht.

21. Verfahren nach einem der Ansprüche 1-20, wobei die zweite Temperatur ausreichend höher ist als die erste Temperatur, um zu ermöglichen, dass die *Bacillus*-Wirtszelle Hyaluronsäure mit einem gewünschten mittleren Molekulargewicht in einem Bereich produziert, der aus der Gruppe der Molekulargewichtsbereiche ausgewählt ist bestehend aus 300 - 350 kDa, 350-400 kDa, 400-450 kDa, 450-500 kDa, 500-550 kDa, 550-600 kDa, 600-650 kDa, 650-700 kDa, 700-750 kDa, und 750-800 kDa.

## Revendications

1. Procédé de production d'un acide hyaluronique avec une masse moléculaire moyenne basse par une fermentation à température contrôlée, le procédé comprenant les étapes consistant à :
(a) cultiver une cellule hôte de *Bacillus* recombinante à une première température conduisant à sa croissance, où la cellule hôte de *Bacillus* comprend une construction d'acide nucléique comprenant une séquence codant une hyaluronane synthase liée de manière opérationnelle à une séquence promoteur étrangère à la séquence codant la hyaluronane synthase ;
(b) cultiver ensuite la cellule hôte de *Bacillus* recombinante de l'étape (a) à une seconde température supérieure à la première température de l'étape (a) dans des conditions appropriées à la production de l'acide hyaluronique, où la seconde température se situe dans la plage de 40 C - 52 °C et est au moins 5 °C supérieure à la première température, moyennant quoi la cellule hôte de *Bacillus* produit de l'acide hyaluronique avec une masse moléculaire moyenne souhaitée dans la plage choisie dans le groupe des masses moléculaires moyennes constitué de 300 - 400 kDa, 400 - 500 kDa, 500 - 600 kDa, 600 - 700 kDa, 700 - 800 kDa ; et
(c) récupérer l'acide hyaluronique.

2. Procédé selon la revendication 1, dans lequel la séquence codant la hyaluronane synthase code pour une hyaluronane synthase du groupe 1.

3. Procédé selon la revendication 1, dans lequel la séquence codant la hyaluronane synthase code pour une hyaluronane synthase du groupe II.

4. Procédé selon l'une quelconque des revendications 1 à 3, dans lequel un sucre précurseur de l'acide hyaluronique est fourni ou produit par la cellule hôte de *Bacillus.*

5. Procédé selon la revendication 4, dans lequel le sucre précurseur est codé par des gènes endogènes, par des gènes non endogènes, ou par une combinaison de gènes endogènes et non endogènes dans la cellule hôte de *Bacillus.*

6. Procédé selon l'une quelconque des revendications 1 à 5, dans lequel la construction d'acide nucléique comprend en outre un ou plusieurs gènes codant pour des enzymes dans la biosynthèse d'un sucre précurseur de l'acide hyaluronique ou la cellule hôte de *Bacillus* comprend en outre une ou plusieurs secondes constructions d'acide nucléique comprenant un ou plusieurs gènes codant pour des enzymes dans la biosynthèse d'un sucre précurseur de l'acide hyaluronique.

7. Procédé selon la revendication 6, dans lequel les un ou plusieurs gènes sont choisis dans le groupe constitué par un gène de l'UDP-glucose 6-déshydrogénase, gène de l'UDP-glucose pyrophosphorylase, gène de l'UDP-N-acétylglucosamine pyrophosphorylase, gène de la glucose-6-phosphate isomérase, gène de l'hexokinase, gène de la phosphoglucomutase, gène de l'amidotransférase, gène de la mutase et gène de l'acétyl-transférase.

8. Procédé selon la revendication 7, dans lequel le gène de l'UDP-glucose 6-déshydrogénase est un gène *hasB* ou un gène *tuaD;* ou des homologues de ceux-ci.

9. Procédé selon la revendication 7, dans lequel le gène de l'UDP-glucose pyrophosphorylase est un gène *hasC* ou un gène *gtaB ;* ou des homologues de ceux-ci.

10. Procédé selon la revendication 7, dans lequel le gène de l'UDP-N-acétylglucosamine pyrophosphorylase est un gène *hasD* ou *gcaD;* ou des homologues de ceux-ci.

11. Procédé selon la revendication 7, dans lequel le gène de la glucose-6-phosphate isomérase est un gène *hasE* ou un homologue de celui-ci.

12. Procédé selon l'une quelconque des revendications 6 à 11, dans lequel les un ou plusieurs gènes codant pour un précurseur de sucre sont sous le contrôle d'un/de précurseur(s) identique(s) ou différent(s) de la séquence codant la hyaluronane synthase.

13. Procédé selon la revendication 12, dans lequel la/les une ou plusieurs séquence(s) promoteur(s) identique(s) ou différente(s) est/sont un promoteur tandem où chaque promoteur du promoteur tandem est lié de manière opérationnelle à la séquence codant la hyaluronane synthase.

14. Procédé selon l'une quelconque des revendications 1 à 13, dans lequel la construction d'acide nucléique comprend en outre une séquence de traitement/stabilisation d'ARNm localisée en aval de la séquence promoteur et en amont de la séquence codant la hyaluronane synthase.

15. Procédé selon la revendication 14, dans lequel la construction d'acide nucléique comprend en outre une séquence de traitement/stabilisation d'ARNm localisée en aval d'un promoteur différent ou de promoteurs différents des un ou plusieurs gènes codant pour des enzymes dans la biosynthèse du sucre précurseur et en amont des un ou plusieurs gènes.

16. Procédé selon l'une quelconque des revendications 1 à 15, dans lequel la cellule hôte de *Bacillus* est choisie dans le groupe constitué de *Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis* et *Bacillus thuringiensis.*

17. Procédé selon l'une quelconque des revendications 1 à 16, dans lequel la construction d'acide nucléique comprend un gène de hyaluronane synthase, gène de l'UDP-glucose 6-déshydrogénase, et gène de l'UDP-glucose pyrophosphorylase lié de manière opérationnelle à un promoteur « consensus » court de *amyQ* ayant la séquence TTGACA pour la région « -35 » et TATAAT pour la région « -10 ».

18. Procédé selon l'une quelconque des revendications 1 à 17, dans lequel la cellule hôte de *Bacillus* contient un gène *cypX* et/ou *yvmC* rompu ou délété.

19. Procédé selon l'une quelconque des revendications 1 à 18, dans lequel la première température se situe dans la plage de 30°C - 40°C.

20. Procédé selon l'une quelconque des revendications 1 à 19, dans lequel l'étape de culture à la seconde température prend jusqu'à au moins 20 % du temps total de 1 culture.

21. Procédé selon l'une quelconque des revendications 1 à 20, dans lequel la seconde température est suffisamment supérieure à la première température pour permettre à la cellule hôte de *Bacillus* de produire de l'acide hyaluronique avec une masse moléculaire moyenne souhaitée dans une plage choisie dans le groupe des plages de masses moléculaires constitué de 300 - 350 kDa, 350 - 400 kDa, 400 - 450 kDa, 450 - 500 kDa, 500 - 550 kDa, 550 - 600 kDa, 600 - 650 kDa, 650 - 700 kDa, 700 - 750 kDa, 750 - 800 kDa.
